Europäisches Patentamt

**European Patent Office**

Office européen des brevets

(19)

(11) Publication number : **0 041 175 B2**

(12) # NEW EUROPEAN PATENT SPECIFICATION

(45) Date of publication of the new patent specification : **09.06.93 Bulletin 93/23**

(51) Int. Cl.⁵ : **G01N 33/84, G01N 33/52**

(21) Application number : **81103793.6**

(22) Date of filing : **25.05.81**

(54) **Ion specific analytical element.**

(30) Priority : **27.05.80 US 153883**

(43) Date of publication of application : **09.12.81 Bulletin 81/49**

(45) Publication of the grant of the patent : **21.11.85 Bulletin 85/47**

(45) Mention of the opposition decision : **09.06.93 Bulletin 93/23**

(84) Designated Contracting States : **BE CH DE FR GB IT LI NL SE**

(56) References cited :
**EP-A- 0 010 615**
**EP-A- 0 019 786**
**EP-A- 0 033 707**
**BE-A- 705 160**

(56) References cited :
**FR-A- 2 069 713**
**US-A- 3 856 649**
**US-A- 4 042 335**
**RESEARCH DISCLOSURE, volume 161, September 1977 Nr. 16113, pages 29-39 "Ion-selective electrode"**

(73) Proprietor : **MILES INC.**
**1127 Myrtle Street**
**Elkhart Indiana 46514 (US)**

(72) Inventor : **Charlton, Steven C.**
**1533 Strong Ave.**
**Elkhart Indiana 46514 (US)**

(74) Representative : **Hansen, Bernd, Dr.rer.nat. et al**
**Hoffmann, Eitle & Partner Patentanwälte**
**Arabellastrasse 4 Postfach 81 04 20**
**W-8000 München 81 (DE)**

EP 0 041 175 B2

## Description

The present invention relates to the field of diagnostic test methods and elements, such as are useful in manual and automated systems, and, more particularly, to those tests useful in qualitative and quantitative determination of the concentration of ions, such as potassium and sodium.

Numerous articles have been published which report the relationship of ions and ionophores as to their electromotive characteristics and the consequent determination of ionic activity by electrode devices.

When two solutions having different ion concentrations are placed in contact, a potential or electromotive force (EMF) is developed which is proportional to:

$$\log \frac{(\text{Ion activity})1}{(\text{Ion activity})2}$$

The two solutions can be separated by a barrier preventing mixing, but allowing electrical contact, e.g. a frit. Alternatively, a membrane containing an ionophore, such as valinomycin, can be used. The properties of the membrane are such that, in the absence of the ionophore, the resistance is so high that it behaves like an open circuit (not enough current flows to drive the voltmeter) and no EMF can be measured. In the presence of potassium ($K^+$), valinomycin (V) produces a conductive path across the membrane by binding $K^+$, thus allowing a small current to flow. Such a system can be illustrated as follows:

|  |  | Membrane, phase 2 |  | $K^+$ |  |
|---|---|---|---|---|---|
| $Cl^-$ $K^+$ | | V | | $Cl^-$ | $Cl^-$ |
| $K^+$ $Cl^-$ | | | | | $K^+$ |
| Test solution phase 1 | $Cl^-$ | | V | $K^+$ | Reference solution, |
| | $K^+$ | | | | phase 3 |
| | $K^+$ | V | | | |
| $Cl^-$ | | | | | |
| $K^+$ | | | | | |
| | $Cl^-$ | | | $Cl^-$ | |

A reference concentration of $K^+$ (Phase 3) exists on one side of the membrane and the EMF developed is measured and used to calculate the unknown concentration from Nernst's equation. Because $K^+$ is the only cation bound to valinomycin, the conductive path only appears for $K^+$ ion and the EMF developed is dependent only upon the $K^+$ concentration gradient across the membrane. The current flowing is so tiny that no significant quantity of $K^+$ or counterion are transported through the membrane. A major difficulty in the use of such ion-selective electrodes has been their tendancy to deteriorate in the accuracy and speed of their response over time. Further, their reading is interpreted by a logarithmic function such that small changes in ion concentration require sophisticated voltmeter equipment.

It has been known that macrocyclic antibiotics such as described above have an effect on the electrical properties of phospholipid bilayer membranes, biological membranes, such that these antibiotics solubilize cations within the membrane, in the form of mobile charged complexes, thereby providing a "carrier" mechanism by which cations can cross the insulating hydrocarbon interior of the membrane. Such complexes have the sole purpose of carrying the charge of the complex through the membrane such that a voltage differential can be determined between solutions on either side of the membrane.

In another field of study, Eisenman et al., J. Membrane Biol. 1:294-345 (1969) discloses the selective extraction of cations from aqueous solutions into organic solvents by macrotetralide actin antibiotics. The experiments involve merely shaking an organic solvent phase containing the antibiotics with aqueous solutions containing various cationic salts of a lipid soluble colored anion. The intensity of color of the organic phase is then measured spectrophotometrically to indicate how much salt has been extracted. Eisenman does not attempt to measure the ion concentration in the aqueous phase. Phase transfer has also been studied by Dix *et al.*, Angew. Chem. Int. Ed. Engl. 17:857 (1978) and in reviews including Burgermeister *et al.*, Top. Curr. Chem. 69:91 (1977); Yu et al., Membrane Active Complexones, Elsevier, Amsterdam (1974); and Duncan, Calcium in Biological Systems, Cambridge University Press (1976).

Partitioning of a compound is rapid and effective between liquids, as shown by Eisenman, because of their mobile nature which allows the transported compound to diffuse rapidly away from the interface. Such a mechanism is normally impossible in the solid phase which is characterized by rigidity, immobility and essentially zero diffusion.

Therefore, there remains the absence of any solid state instrument or device by which the concentration of ions in a solution is photometrically determined. Even with expensive and cumbersome equipment no visual measurement of ion concentration has been available using solid state analytical techniques. Thus specific solid state determinations of ion concentration, suitable for clinical chemical use, have heretofore been unavailable.

The present invention provides an entirely new concept for the determination of an ion in a liquid sample which for the first time allows for the quantitative analysis of a specific ion in the sample by the simple dip and read method, the accuracy of the analysis not being affected by the presence of otherwise interfering ions. The invention is based on the finding that if a nonpolar material which is incorporated with an ionophore like valinomycin, is contacted with an aqueous sample containing various ions, one of these ions ($K^+$ in the case of valinomycin) despite of its polar nature may be selectively transferred into the nonpolar material by means of an active transport phenomenon caused by the ionophore. The amount of ion transferred into the nonpolar material is correlated to its concentration in the liquid sample. The ion in the nonpolar material may then be quantitatively determined by means of a detectable species (e.g. a counterion dye substance) which is capable of interacting with the ion to produce a detectable response.

US-A-4 042 335 is concerned with the problem of uniformly distributing a liquid sample in a test device. Ions are not even mentioned as possible analytes. In contrast to the present invention, the reagent layer of the test device according to US-A-4 042 335 is permeable to the liquid sample and for that reason is made of a porous and/or hydrophilic material. Reagent layers of this kind would be unsuitable for the purpose of the present invention since they would not allow for the selective separation of a specific ion from a liquid sample.

EP-A-0 010 615 is using another concept for the determination of ions of other polar substances which does not avoid interferences by similar substances as the analyte to be determined. According to EP-A-0 010 615 the ionophores are covalently linked to a chromogen which changes its extinction when the complex of ion and ionophor is formed. While in the specification the incorporation of a dyestuff salt into valinomycin is discussed so that the dyestuff is forced out when the potassium from the sample is complexed, the publication is silent, however, on the essential features of the present invention and gives no hint how to overcome the problem of interference by other ions such as sodium.

Fig. 1 is a graphical representation of the data using tris-2-ethylhexylphosphate as plasticizer, reported in Table 4.

Fig. 2 is a graphical representation of the data using di-2-ethylhexylsebacate as plasticizer, reported in Table 4.

Fig. 3 is a graphical representation of the data using Phloxine B as the counterion, reported in Table 5.

Fig. 4 is a graphical representation of the data using Orange IV as the counterion, reported in Table 5.

Fig. 5 is a graphical representation of the data, using Eosin Y as the counterion, reported in Table 5.

Fig. 6 is a graphical representation of the data, using DCPIP as the counterion, reported in Table 6.

Summary of the invention

In accordance with the present invention there is provided a method for determination of an ion in a liquid sample and a test device or analytical element for use in such determinations as claimed in claims 1, 8 and 10. The contemplated method comprises contacting such an analytical element with the sample in the presence of a detectable species associated with said ionic analyte, said analytical element comprising a layer of substantially nonpolar material incorporated with an analyte-specifc ionophore, and photometrically measuring the amount of said detectable species which has migrated to at least a surface portion of said analytical element, the amount of said detectable species which has migrated being indicative of the concentration of the specific ionic analyte in the sample. The reacted element is stable and the readings uniformly reliable over at least a period of days such that, for example, a number of reacted elements can be collected for reading at one time.

The solid state test device or analytical element of the present invention comprises a layer comprising a substantially nonpolar material incorporated with an ionophore capable of selectively forming a complex with the ion to be termined. In a particular embodiment of the present invention, the test device comprises an integral analytical element comprising a layer of substantially nonpolar material incorporated with an analyte-specific ionophore and further including, in laminar relationship therewith, a second film layer incorporated with a detectable species associated with said ionic analyte. Reference to a laminar relationship between an ionophore layer and any additional layer(s) connotes the ability of a fluid, whether liquid or gaseous, to pass between superposed surfaces of such layers. Such layers can be contiguous or separated by intervening layers. Any intervening layer will be such as does not prevent passage between all layers. The analytical element can be self-supporting or carried on a support, such as a support that can transmit electromagnetic radiation of one or more wavelengths within the region between about 200 nanometers (nm) and about 900 nm.

As stated above, one aspect of the invention is a method for determining the concentration of a specific ionic analyte in a liquid sample, which method comprises contacting an analytical element with the sample in the presence of a detectable species associated with said ionic analyte, said analytical element comprising a layer of substantially nonpolar material incorporated with an analyte-specific ionophore; and photometrically measuring the amount of said detectable species which has migrated to at least a surface portion of said analytical element, the amount of said detectable species which has migrated being indicative of the concentration of the specific ionic analyte in the sample. After said contacting and before said measuring said method can optionally comprise the additional step of removing any excess sample from said analytical element. Photometric measurement is contemplated to include colorimetric and fluorometric measurement.

In one embodiment the step of contacting comprises adding to said liquid sample a detectable species which is associated with said ionic analyte and then contacting said analytical element with the detectable species containing sample. In another embodiment said contacting comprises contacting an analytical element with the sample, said analytical element comprising a layer of substantially nonpolar material incorporated with an analyte-specific ionophore; and, in laminar relationship therewith, a second layer incorporated with a detectable species associated with said ionic analyte.

In one example of the multilayer element described above the ionophore layer has a pair of opposite surfaces, said detectable species layer being in laminar relationship with one of said pair of surfaces and said contacting comprises contacting the other of said surfaces. In another example the detectable species layer has a pair of opposite surfaces, said ionophore layer being in laminate relationship with one of said pair of surfaces and said contacting comprises contacting the other of said surfaces.

The substantially nonpolar material of said film layer can be a composition comprising a plasticizer and a nonpolar polymer suitable to impart dimensional stability. Suitable plasticizers can include the phthalates, such as di-pentylphthalate, the sebacates, such as di-2-ethylhexyl sebacate, and the phosphates, such as tris-2-ethylhexyl phosphate. Suitable nonpolar polymers, such as polyvinyl chloride and polyurethane, can include those useable to form blush polymer layers.

The ionophore/ion pairs to which the present invention is applicable include any of those known to the art and any for which the relationship may subsequently be observed. Examples of such pairs include:

valinomycin/$K^+$;

4, 7, 13, 16, 21-pentaoxa-1,10-diazabicyclo-(8,8,5)tricosane [Kryptofix® 221]/$Na^+$;

4, 7, 13, 16, 21, 24-hexaoxa-1,10-diazabicyclo-(8,8,8)hexacosane [Kryptofix® 222]/$K^+$; and

4, 7, 13, 18-tetraoxa-1,10-diazabicyclo(8,5,5)eicosane [Kryptofix® 211]$Li^+$.

Kryptofix is a registered trademark of MCB Reagents, associate of E. Merck, 2902 Highland Avenue, Cincinnati, Ohio.

The detectable species is preferably a counter ion which is, for example, a chromophore or fluorophore. A suitable chromophore is dichlorophenolindophenol. Suitable fluorophores include fluoroscein and its derivatives, 8-anilino-1-naphthalenesulfonic acid, Erythrosin B and 7-amino-4-trifluoromethyl coumarin.

Such a counterion could likewise be associated with another component which is interreactive with a reagent which has been incorporated into the ionophore layer. Due to the presence of an interactive (e.g. chemically reactive) material, and a uniform apparent concentration of substance provided to the ionophore layer, a uniform, quantitative detectable change can be produced in the element. Such a change, which can be the generation or destruction of coloration or fluorescence, can be detected quantitatively by radiometric techniques and, if desired, by automatic radiometric sensing devices such as photometric devices.

In preparing integral analytical elements of this invention, the layers can be preformed separately and laminated to form the overall element. Layers prepared in such a manner are typically coated from solution or dispersion on a surface from which the dried layer can be physically stripped. However, a convenient method which can avoid problems of multiple stripping and lamination steps is to coat an initial layer on a stripping surface or a support, as desired, and thereafter to coat successive layers directly on those coated previously. Such coating can be accomplished by hand, using a blade coating device, or by machine, using techniques such as dip or bead coating. If machine coating techniques are used, it is often possible to coat adjacent layers simultaneously, using hopper coating techniques well known in the preparation of light-sensitive photographic films and papers.

Blush polymer layers, referred to above, can be formed on a substrate by dissolving a polymer in a mixture of two liquids, one of which is of a lower boiling point and is a good solvent for the polymer and the other of which is of a higher boiling point and is a non-solvent or at least a poor solvent for the polymer. Such a polymer solution is then coated on the substrate, and dried under controlled conditions. The lower boiling solvent evaporates more readily and the coating can become enriched in the liquid which is a poor solvent or non-solvent. As evaporation proceeds, under proper conditions, the polymer forms as a porous layer. Many different polymers can be used, singly or in combination, for preparing porous blush polymer layers for use in this invention.

Typical examples include polycarbonates, polyamides, polyurethanes and cellulose esters such as cellulose acetate. For layers such as those containing an ionophore or detectable species, a coating solution or dispersion including the matrix and incorporated active materials can be prepared, coated as discussed herein and dried to form a dimensionally stable layer.

The thickness of any layer and its degree of permeability are widely variable and depend on actual usage. Dry thicknesses of from about 5 μm to about 100 μm have been convenient, although more widely varying thickness may be preferable in certain circumstances. For example, if comparatively large amounts of interactive material, e.g. polymeric materials like enzymes, are required, it may be desirable to use slightly thicker layers.

It can also be desirable to include within an element one or more reflective layers, optionally absorptive to detecting radiation, such as to facilitate result detection by reflection radiometry, e.g. reflection photometry or a similar technique. Such reflectance can be provided by one of the above-described layers or it can be provided by an additional layer that may not have an additional function within the element. Pigments, such as titanium dioxide and barium sulfate, which are reflective can be used to advantage in a reflecting layer. Blush polymers can also constitute a suitable reflecting material. In one preferred aspect, blush polymer layers can also incorporate a pigment to enhance reflectivity or other functions. The amount of pigment that can be included in a layer together with a blush polymer is highly variable, and amounts of from about 1 to about 10 parts by weight of pigment per part by weight of blush polymer are preferred, with from about 3 to about 6 parts pigment per part of blush polymer being most preferred.

In the layers of the element, it can be advantageous to incorporate one or more surfactant materials such as anionic and nonionic surfactant materials. They can, for example, enhance coatability of layer formulations and enhance the extent and range of wetting in layers that are not easily wetted by liquid samples in the absence of an aid such as a surfactant. In layers of the element it can also be desirable to include materials that can render non-active in the analysis of choice, by chemical reaction or otherwise, materials potentially deleterious to such analysis.

As mentioned previously herein, the integral analytical elements can be self-supporting or coated on a support. Useful support materials include paper and polyolefin coated paper, as well as a variety of polymeric materials such as cellulose acetate, poly(ethylene terephthalate), polycarbonates and polyvinyl compounds such as polystyrenes, etc. The support can be opaque or it can transmit light or other energy. A support of choice for any particular element will be compatible with the intended mode of result detection. Preferred supports include transparent support materials capable of transmitting electromagnetic radiation of a wavelength within the region between about 200 nm and about 900 nm. The support need not, of course, transmit over the entire 200-900 nm region, although for fluorometric detection of analytical results through the support it is desirable for the support to transmit over a wider band or, alternatively, to transmit at the absorption and emission spectra of the fluorescent materials used for detection. It may also be desirable to have a support that transmits one or more narrow wavelength bands and is opaque to adjacent wavelength bands. This could be accomplished, for example, by impregnating or coating the support with one or more colorants having suitable absorption characteristics.

As can be appreciated, a variety of different elements, depending on the analysis of choice, can be prepared in accordance with the present invention. Elements can be configured in a variety of forms, including elongated tapes of any desired width, sheets or smaller chips. Particular elements can be adapted for one or more tests of a single type or a variety of tests of differing types. In such later event, it can be desirable to coat a common support with one or more strips or channels, each optionally of a different composition to form a composite element suited for conducting a variety of desired tests.

Integral analytical elements of the present invention can be adapted for use in carrying out a wide variety of chemical analyses, not only in the field of clinical chemistry but in chemical research and in chemical process control laboratories. They are well suited for use in clinical testing of body fluids, such as blood, blood serum and urine, since in this work a large number of repetitive tests are frequently conducted and test results are often needed a very short time after the sample is taken. In the field of blood analysis, for example, the multilayer element can be adapted for use in carrying out quantitative analysis for many of the ionic blood components which are routinely measured.

The present elements are placed in use by applying to the element a sample of liquid under analysis. In a typical analytical procedure using the present elements, which could be manual or automated, the element is taken from a supply roll, chip packet or other source and positioned to receive a free drop, contact spot or other form of liquid sample, such as from an appropriate dispenser. After sample application, and desirably after the liquid sample has been taken up by the layer to which it has been contacted, the element is exposed to any conditioning, such as heating, humidification or the like, that may be desirable to quicken or otherwise facilitate obtaining the desired test result. If an automated procedure is used, it can also be desirable to have the element accomplish its function within several seconds. This can be accomplished conveniently by appropriate selection of various parameters, such as layer thickness, void volume in porous layers, etc.

After the analytical result is obtained as a detectable change, it is measured, usually by passing the element through a zone in which suitable apparatus for reflection, transmission or fluorescence photometry is provided. Such apparatus would serve to direct a beam of energy, such as light, through, in one embodiment, the support. The light is then reflected from the element back to a detecting means or passes through the element to a detector in the case of transmission detection. In a preferred mode, the analytical result is detected in a region of the element totally within the region in which such result is produced. Use of reflection spectrophotometry can be advantageous in some situations as it effectively avoids optical interference from any residues, such as blood cells, which have been left on or in the layers of the element. Conventional techniques of fluorescence spectrophotometry can also be employed if desired. Furthermore, when blood serum is tested or means are provided for eliminating unwanted whole blood residues, transmission techniques can be used to detect and quantify the indicating reaction products by reacting a flow of radiant energy, for example, ultraviolet, visible or infrared radiation at one surface of the element and measuring the output of that energy from the opposing surface of the element. Generally, electromagnetic radiation in the range of from about 200 to about 900 nm has been found useful for such measurements, although any radiation to which the element is permeable and which is capable of quantifying the product produced in the element can be used. Various calibration techniques can be used to provide a control for the analysis. As one example, a sample of analyte standard solution can be applied adjacent to the area where the drop of sample is placed in order to permit the use of differential measurements in the analysis.

Example I

In the experiment described by this example, an integral analytical element was prepared and tested for its ability to quantitatively determine, as read by reflectance, the presence of potassium in a liquid sample.

Element preparation

The solution used in preparing the potassium sensitive element contained the following components:

| Component | Quantity | Final concentration (g/100 ml solution) |
|---|---|---|
| polyvinylchloride (PVC) | 38.4 mg | 3.84 |
| dipentylphthalate (DPP) | 129.6 mg | 12.96 |
| Valinomycin | 4.0 mg | 0.4 |
| tetrahydrofuran (THF) | 0.87 ml | 89.0 |
| Total value | 1.0 ml | 100 ml |

One ml of this solution was placed on a transparent polyester film, Gel Bond™ (Marine Colloids, Inc.), uncoated side and spread to a depth of 0.254 mm using a conventional doctor blade. Scotchpar 70 GAB 25 (3M Corporation) can be used in lieu of Gel Bond. Approximately 30 cm$^2$ of 25 μm thickness were produced. The film was air dried for approximately 30 minutes.

For reflectance measurements the polyester film was then cut into 1 cm segments and the uncoated side faced onto a light scattering polystyrene support (white Trycite®) with 3M brand double faced adhesive tape. The segments were then cut to produce a 1 x0.75 cms reactive element composed of a transparent potassium sensitive ionophore layer on a polyester support with a light scattering background.

Test solution

Potassium chloride was added to aliquots of a solution containing 35.6 mM tris-Cl and 8.89 mM Erythrosin B (pH 7.2) to give potassium concentrations of 0.133, 0.2, 0.3, 0.4, 0.5, 0.6, 0.75, 0.9 and 1.1, respectively. A solution was also made which contained no potassium.

Analytical procedure

Analysis of each aliquot was performed by placing sufficient test solution to produce a dome of liquid on the element. After four (4) minutes the liquid was removed by a gentle stream of water and the element gently blotted dry with paper tissue. The retained dye was quantified from the percent reflectance (%R) at 530 nanometers (nm) as measured by a reflectance spectrometer. Readings taken on test solutions of 0.2 and 0.5 mM potassium concentrations were used to calibrate the spectrometer.

Results

The readings obtained by this analytical procedure were in the form of %R units which were mathematically converted, by the Kubelka-Monk equation and the calibration values, to potassium concentration ($K^+$) units, these readings and respective observed concentrations having been as reported in Table 1.

TABLE 1

| Actual $K^+$ (mM) | (%R) | Observed $K^+$ (mM) |
|---|---|---|
| 0 | 62.9 | 0.03 |
| 0.133 | 40.5 | 0.19 |
| 0.2 | 36.2 | 0.24 |
| 0.3 | 30.7 | 0.35 |
| 0.4 | 27.1 | 0.44 |
| 0.5 | 24.9 | 0.51 |
| 0.6 | 23.0 | 0.58 |
| 0.75 | 19.4 | 0.76 |
| 0.9 | 18.4 | 0.83 |
| 1.1 | 15.9 | 1.02 |

Conclusion

The resultant data shows the integral analytical element to provide a quantitative detectable response to the potassium concentration of each of the aliquots tested.

Example II

This example illustrates that the element containing the ionophore valinomycin is selective for potassium and not responsive to sodium, thus providing a differential test, in this experiment read by absorbance, of these commonly occurring ions in biological fluid.

Element preparation

The potassium specific element contained the same components and was prepared as described in Example I, with the exception that it was not fixed to a reflective support.

Test solutions

Potassium chloride and sodium chloride were added to aliquots of a solution containing 5 mM Erythrosin B and 50 mM tris-acetate (pH 7.2) to give potassium and sodium concentrations as set forth in Table 2.

EP 0 041 175 B2

TABLE 2

| Solution | $K^+$ (mM) | $Na^+$ (mM) |
|---|---|---|
| 1 | 0 | 4 |
| 2 | 0 | 10 |
| 3 | 0 | 20 |
| 4 | 0 | 50 |
| 5 | 0 | 100 |
| 6 | 2.2 | 0 |

Analytical procedure

The analysis of the above prepared test solutions was performed by the same procedure used in Example I, with the exceptions that the retained dye was quantified from the absorbance, rather than reflectance, and that readings were taken at 553 nm. Three measurements were made at each level and the mean determined as reported below.

Results

The readings obtained by this analytical procedure were in the form of absorbance units which were mathematically converted to potassium concentrations ($K^+$) units, using the first and last entries as calibrating values. These readings and respective observed concentrations were as reported in Table 3.

TABLE 3

| Actual $K^+$ (mM) | Actual $Na^+$ (M) | Absorbance (553 nm) | Observed $K^+$ (mM) |
|---|---|---|---|
| 0 | 4 | 0.063 | 0 |
| 0 | 10 | 0.062 | 0 |
| 0 | 20 | 0.063 | 0 |
| 0 | 50 | 0.060 | −0.01 |
| 0 | 100 | 0.057 | −0.03 |
| 2.2 | 0 | 0.547 | 2.2 |

Conclusion

The resultant data shows the integral analytical element to provide a quantitative detectable response specifically to the potassium concentration of each of the aliquots tested, irrespective of sodium ion concentration.

Example III

In the experiment described by this example a substitution of plasticizers used in preparation of the ionophore layer was made.

Element preparation

The solution used in preparing the potassium specific element contained the following components:

8

| Component | Quantity | Final concentration (g/100 mls solution) |
|---|---|---|
| PVC | 40 mg | 4 |
| Plasticizer | 100 mg | 10 |
| Valinomycin | 1.5 mg | 0.15 |
| THF | 0.9 ml | 90 |
| Total Value | 1 ml | 100 ml |

Plasticizers used were tris-2-ethylhexylphosphate (TEP) and di-2-ethylhexyl sebacate (DHS). The element was prepared by the procedure described in Example I.

Test solutions

Potassium chloride was added to aliquots of a solution containing 40 mM tris-Cl and 10 mM Erythrosin B (pH 7.2) to give potassium concentrations of 0.2, 0.4, 0.6, 0.8 and 1.1 mM. One aliquot was prepared containing no potassium.

Analytical procedure

The procedure was the same as in Example I, except that the wavelength of measurement was 540 nm and calibration levels were 0.4 and 0.8 mM potassium. Tests were performed in triplicate and the mean determined as reported below.

Results

The mean reflectance readings and observed concentrations derived therefrom are reported in Table 4.

TABLE 4

| Actual $K^+$ (mM) | TEP | | DHS | |
|---|---|---|---|---|
| | %R | Observed $K^+$ (mM) | %R | Observed $K^+$ (mM) |
| 0 | 39.8 | −0.23 | 62.8 | 0 |
| 0.2 | 33.0 | 0.18 | 41.7 | 0.21 |
| 0.4 | 29.4 | 0.47 | 34.1 | 0.37 |
| 0.6 | 27.5 | 0.67 | 28.4 | 0.55 |
| 0.8 | 25.9 | 0.86 | 24.4 | 0.74 |
| 1.1 | 24.3 | 1.07 | 20.3 | 1.02 |

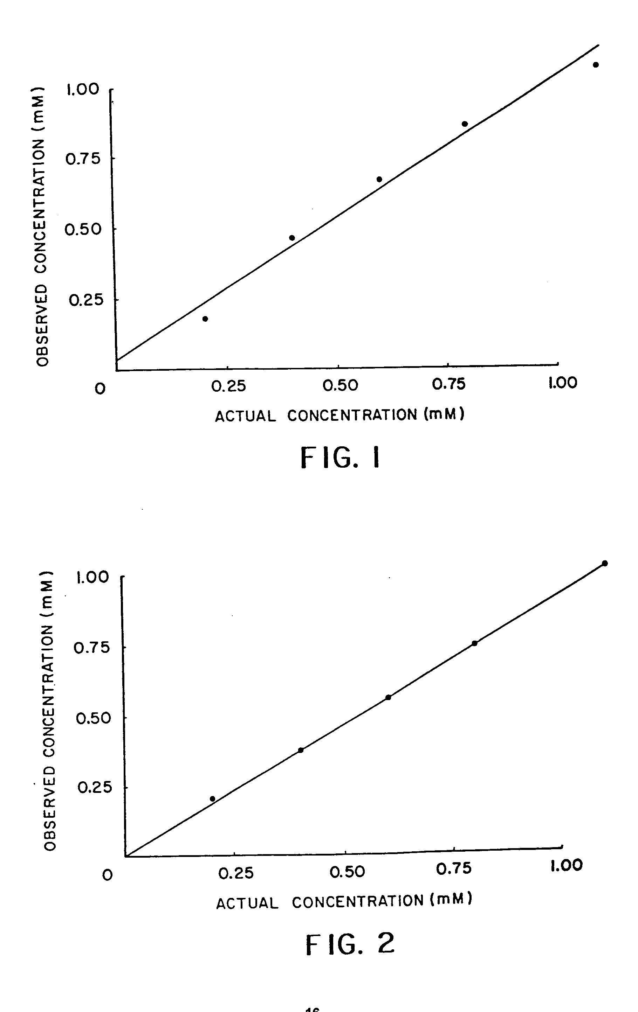

Graphical representations of the data reported in Table 4 for TEP and DHP are provided as Figs. 1 and 2, respectively.

Conclusion

The resultant data shows that elements prepared to contain either of the above plasticizers were effective to quantitatively detect the concentration of potassium in a sample with accuracy.

## Example IV

In this example a selection of alternate dyes was used to show the wide variety of detectable species which are suitable for use with the invention.

### Element preparation

The potassium specific element contained the same components and was prepared as described in Example III, with the exception that the plasticizer dipentylphthalate was used.

### Test solution

Four test solutions were prepared as described in Example III except that, instead of Erythrosin B, each solution contained one of the alternate dyes 2,6-Dichlorophenol indophenol (DCPIP), Orange IV, Phloxine B and Eosin Y.

### Analytical procedure

The analytical procedure was the same as Example III with the exception that compositions containing the various dyes were read at the following wavelengths:

Phloxine B (550 nm)     Orange IV (430 nm)

DCPIP (680 nm)     Eosin Y (550 nm)

### Results

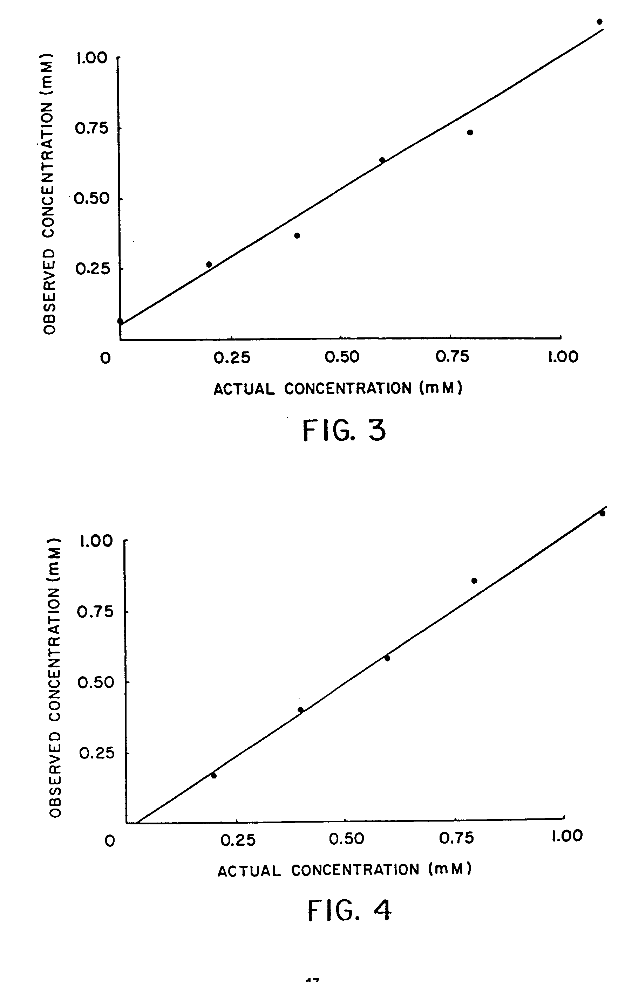

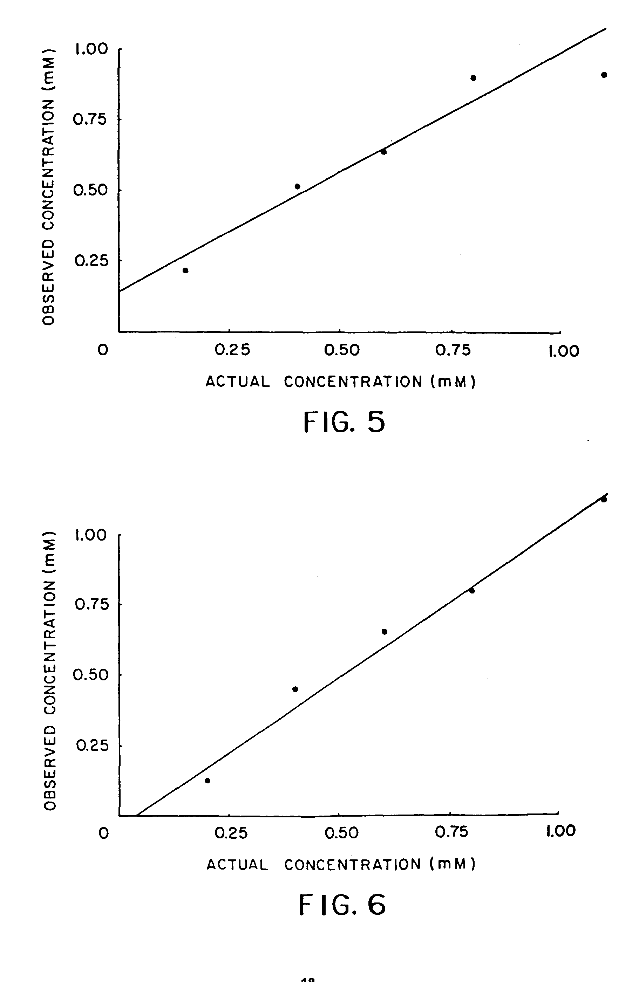

The reflectance readings, along with the potassium concentrations calculated from them are shown in Table 5. Graphical representations of the correlation between actual and observed concentrations for test solutions containing Phloxine B, Orange IV, Eosin Y and DCPIP are provided as Figs. 3, 4, 5 and 6, respectively.

TABLE 5

| Actual $K^+$ (mM) | Phloxine B | | DCPIP | | Orange IV | | Eosin Y | |
|---|---|---|---|---|---|---|---|---|
| | %R | $K^+$ (mM) | %R | $K^+$ (mM) | %R | $K^+$ (mM) | %R | $K^+$ (mM) |
| 0 | 59.7 | 0.06 | 52.3 | −0.16 | 65.1 | −0.1 | 64.7 | 0.66 |
| 0.2 | 57.0 | 0.26 | 35.3 | 0.10 | 49.9 | 0.17 | 60.9 | 0.21 |
| 0.4 | 55.7 | 0.36 | 25.5 | 0.45 | 42.5 | 0.41 | 59.4 | 0.51 |
| 0.6 | 52.7 | 0.63 | 22.0 | 0.66 | 38.8 | 0.57 | 58.8 | 0.63 |
| 0.8 | 51.6 | 0.74 | 20.3 | 0.79 | 34.0 | 0.84 | 57.6 | 0.90 |
| 1.1 | 48.1 | 1.13 | 17.0 | 1.12 | 30.9 | 1.08 | 57.5 | 0.91 |

### Conclusion

The resultant data shows that elements prepared to contain any of the above detectable species are effective to quantitatively detect the concentration of potassium in a sample with accuracy.

## Example V

This example shows the analytical element had a quantitative and specific response to potassium when a different ionophore (Kryptofix® K 222) was used.

## Element preparation

The solution used in preparing the potassium specific element contained the following components:

| Component | Quantity | Final concentration (g/100 mls solution) |
|---|---|---|
| PVC | 38.40 mg | 3.84 |
| DPP | 64.80 mg | 6.48 |
| Kryptofix K222 | 2.40 mg | 0.24 |
| THF | 0.935 ml | 93.50 |
| Total value | 1 ml | 100 ml |

The element was prepared by the procedure described in Example I.

## Test solution

Potassium chloride was added to aliquots of a solution of 5 mM Erythrosin B and 20 mM tris-Cl (pH 7.3) to generate solutions containing 0, 0.1, 0.3, 1.0, 3.0, and 30 mM potassium, respectively. A set of solutions was made in the same way using sodium chloride in lieu of potassium chloride.

## Analytical procedure

The procedure was the same as in Example I, including the wavelength used, except that the calibrating solutions contained 0.1 and 1.0 mM potassium. Tests were performed in triplicate and the means determined as reported below.

## Results

The mean reflectance readings, and observed concentrations derived therefrom, of these elements to the potassium and sodium test solutions are reported in Table 6.

TABLE 6

| Actual $K^+$ (mM) | %R | Observed $K^+$ (mM) | Actual $Na^+$ (mM) | %R | Observed $Na^+$ |
|---|---|---|---|---|---|
| 0 | 56.1 | 0.04 | 0 | 54.6 | 0.03 |
| 0.1 | 50.6 | 0.1 | 0.1 | 53.4 | 0.05 |
| 0.3 | 46.1 | 0.25 | 0.3 | 53.3 | 0.05 |
| 1.0 | 28.1 | 1.0 | 1.0 | 54.3 | 0.06 |
| 3.0 | 9.97 | 5.2 | 3.0 | 51.5 | 0.08 |

## Conclusion

Elements containing an appropriate ionophore, such as Kryptofix K222, ionophore show a specific and quantitative response to potassium ion.

## Example VI

This example shows the analytical element has a specific and quantitative response to sodium ion when

11

the ionophore Kryptofix® K221 is used.

Element preparation and test procedure

The element prepared as in Example V except for the use of Kryptofix K221 in lieu of Kryptofix 222.

Analytical procedure

The procedure was the same as in Example I, including wavelength used, except that calibrating solutions contained 0.1 and 10.0 mM sodium. Tests were performed in triplicate and the means determined as reported below.

Results

The mean reflectance readings, and observed concentrations derived therefrom, of these elements to sodium and potassium test solutions are reported in Table 7.

TABLE 7

| Actual Na$^+$ (mM) | %R | Observed Na$^+$ (mM) | Actual K$^+$ (mM) | %R | Observed K$^+$ (mM) |
|---|---|---|---|---|---|
| 0 | 56.4 | 0.14 | 0 | 54.7 | 0.10 |
| 0.1 | 56.7 | 0.10 | 0.1 | 56.3 | 0 |
| 0.3 | 52.5 | 0.47 | 0.3 | 54.2 | 0.18 |
| 1.0 | 47.8 | 1.4 | 1.0 | 54.7 | 0.10 |
| 3.0 | 30.2 | 8.4 | 3.0 | 57.9 | 0 |
| 10.0 | 28.0 | 10 | 10 | 54.4 | 0.14 |
| 30.0 | 18.6 | 21.5 | 30 | 53.4 | 0.31 |

Conclusion

Analytical elements containing a suitable ionophore, such as Kryptofix K221, shows a quantitative and specific response to sodium ion.

**Claims**

1. A solid state test device for the photometric determination of the concentration of a specific ion in an aqueous test sample in the presence of a detectable species associated with that ion, characterized in that the test device comprises a layer containing a substantially nonpolar material, said nonpolar material being incorporated with an ionophore capable of selectively forming a complex with the specific ion.

2. The test device of claim 1, wherein the nonpolar material is affixed to a support member.

3. The test device of any of claims 1 and 2, wherein the detectable species is a counterion dye substance, preferably Erythrosin B.

4. A test device according to any of claims 1 to 3, wherein the detectable species is a fluorophore, preferably a derivative of fluoresrein, 8-anilino-1-naphthalene sulfonic acid or 7-amino-4-trifluoromethyl coumarin.

5. A test device according to any of claims 1 to 4, wherein the ionophore is valinomycin, 4,7,13,16,21 - pen- taoxa - 1,10 - diaza - bicyclo(8,8,5)tricosane, 4,7,13,16,21,24 - hexaoxa - 1,10 - diazabicyclo(8,8,8)hexacosane or 4,7,13,18 - tetraoxa - 1,10 - diazabicyclo(8,5,5)eicosane, preferably valinomycin.

6. A test device according to any of claims 1 to 5, wherein said nonpolar material comprises a plasticizer and a nonpolar polymer suitable to impart dimensional stability.

7. A solid state test device for the photometric determination of the concentration of a specific ion in an aqueous test sample, characterized in that the test device comprises:
a) a first layer containing a substantially nonpolar material said nonpolar material being incorporated with an ionophore selectively forming a complex with the specific ion; and in laminar relation thereto;
b) a second layer incorporated with a detectable species; and
c) a support member.

8. A method for the photometric determination of the concentration of a specific ion in an aqueous test sample, characterized in that the method comprises the steps of:
a) adding to the test sample a photometrically detectable species associated with the ion, the latter being capable to interact with the complex of ionophore and said ion;
b) contacting the test device of any of claims 1 to 6 with the detectable species containing sample; and
c) photometrically measuring the amount of said detectable species which has migrated at least to the surface portion of the test device.

9. The method of claim 8, characterized in that the excess detectable species containing sample is removed from the device prior to measuring the response.

10. Use of the test device according to any of claims 1 to 7 in the determination of sodium, potassium or lithium.


**Patentansprüche**

1. Festphasen-Testvorrichtung für die photometrische Bestimmung der Konzentration eines speziellen Ions in einer wäßrigen Testlösung in Gegenwart einer nachweisbaren Species, die mit jenem Ion assoziiert ist,
dadurch gekennzeichnet, daß
die Testvorrichtung eine Schicht umfaßt, die ein im wesentlichen unpolares Material enthält, wobei in das unpolare Material ein Ionophor eingemischt ist, der imstande ist, selektiv einen Komplex mit dem speziellen Ion zu bilden.

2. Testvorrichtung nach Anspruch 1, wobei das unpolare Material an einem Trägerelement befestigt ist.

3. Testvorrichtung nach einem der Ansprüche 1 und 2, wobei die nachweisbare Species ein Gegenion-Farbstoff, vorzugsweise Erythrosin B, ist.

4. Testvorrichtung nach einem der Ansprüche 1 bis 3, wobei die nachweisbare Species ein Fluorophor, vorzugsweise ein Derivat von Fluorescein, 8-Anilino-1-naphthalinsulfonsäure oder 7-Amino-4-trifluormethylkumarin, ist.

5. Testvorrichtung nach einem der Ansprüche 1 bis 4, wobei der Ionophor Valinomycin, 4,7,13,16,21-Pentaoxa-1, 10-diazabicyclo-(8,8,5)tricosan, 4,7,13,16,21,24-hexaoxa-1,10-diazabicyclo(8,8,8)hexacosan oder 4,7,13,18-tetraoxa-1,10-diazabicyclo(8,5,5)eicosan, vorzugsweise Valinomycin ist.

6. Testvorrichtung nach einem der Ansprüche 1 bis 5, worin das unpolare Material einen Weichmacher und ein unpolares Polymer, welches geeignet ist, Dimensionsstabilität zu verleihen, umfaßt.

7. Festphasen-Testvorrichtung für die photometrische Bestimmung der Konzentration eines speziellen Ions in einer wäßrigen Testprobe,
dadurch gekennzeichnet, daß die Testvorrichtung
a) eine erste Schicht, die ein im wesentlichen unpolares Material enthält, in das ein Ionophor eingemischt ist, welcher selektiv einen Komplex mit dem speziellen Ion bildet; und in laminarer Beziehung

EP 0 041 175 B2

dazu
b) eine zweite Schicht, in die eine nachweisbare Species eingemischt ist; und
c) ein Trägerelement umfaßt.

8. Verfahren zur photometrischen Bestimmung der Konzentration eines speziellen Ions in einer wäßrigen Testlösung,
dadurch gekennzeichnet, daß das Verfahren folgende Schritte umfaßt:
a) Zu der Testprobe wird eine photometrisch nachweisbare Species, die mit dem Ion assoziiert ist, gegeben, wobei das letztere imstande ist, mit dem Komplex aus Ionophor und dem Ion in Wechselwirkung zu treten;
b) die Testvorrichtung nach einem der Ansprüche 1 bis 6 wird mit der die nachweisbare Species enthaltenden Probe in Kontakt gebracht; und
c) die Menge der nachweisbaren Species, die mindestens zum Oberflächenabschnitt der Testvorrichtung gewandert ist, wird gemessen.

9. Verfahren nach Anspruch 8, dadurch gekennzeichnet, daß der Überschuß an nachweisbarer Species, welche die Probe enthält, vor dem Messen der Reaktion entfernt wird.

10. Verwendung der Testvorrichtung nach einem der Ansprüche 1 bis 7 zur Bestimmung von Natrium, Kalium oder Lithium.

**Revendications**

1. Dispositif d'analyse à l'état solide pour la détermination photométrique de la concentration d'un ion particulier dans un échantillon aqueux à analyser en présence d'une substance détectable associée à cet ion, caractérisé en ce qu'il comprend une couche renfermant une matière principalement non polaire, ladite matière non polaire comportant un ionophore capable de former sélectivement un complexe avec l'ion particulier.

2. Dispositif d'analyse suivant la revendication 1, dans lequel la matière non polaire est fixée à un élément de support.

3. Dispositif d'analyse suivant l'une quelconque des revendications 1 et 2, dans lequel le corps détectable comprend une substance colorante à ion complémentaire, de préférence l'érythrosine B.

4. Dispositif d'analyse suivant les revendications 1 à 3, dans lequel la substance détectable est un fluorophore, de préférence un dérivé de fluorescéine, l'acide 8-anilino-1-naphtalènesulfonique ou la 7-amino-4-trifluorométhylcoumarine.

5. Dispositif d'analyse suivant l'une quelconque des revendications 1 à 4, dans lequel l'ionophore est la valinomycine, le 4,7,13,16,21-pentaoxa-1,10-diazabicyclo [8,8,5]tricosane, le 4,7,13,16,21,24-hexaoxa-1,10-diazabicyclo[8,8,8]hexacosane ou le 4,7,13,18-tétraoxa-1,10-diazabicyclo[8,5,5]eicosane, de préférence la valinomycine.

6. Dispositif d'analyse suivant l'une quelconque des revendications 1 à 5, dans lequel ladite matière non polaire comprend un plastifiant et un polymère non polaire apte à conférer la stabilité dimensionnelle.

7. Dispositif d'analyse à l'état solide pour la détermination photométrique de la concentration d'un ion particulier dans un échantillon aqueux à analyser, caractérisé en ce qu'il comprend :
a) une première couche contenant une matière principalement non polaire à laquelle est incorporé un ionophore formant sélectivement un complexe avec l'ion particulier ; et en relation laminaire avec lui ;
b) une seconde couche à laquelle est incorporée une substance détectable ; et
c) un élément de support.

8. Méthode de détermination photométrique de la concentration d'un ion particulier dans un échantillon aqueux à analyser, caractérisée en ce qu'elle comprend les étapes qui consistent
a) à ajouter à l'échantillon à analyser un corps détectable par photométrie associé à l'ion, ce dernier étant susceptible d'interaction avec le complexe d'un ionophore et de l'ion ;

14

b) à mettre le dispositif d'analyse suivant l'une quelconque des revendications 1 à 6 en contact avec l'échantillon contenant le corps détectable, et

c) à mesurer par photométrie la quantité de corps détectable qui a migré au moins jusqu'à la portion de surface du dispositif d'analyse.

9. Méthode suivant la revendication 8, caractérisée en ce que l'échantillon en excès contenant le corps détectable est enlevé du dispositif avant la mesure de la réponse.

10. Utilisation d'un dispositif d'analyse suivant l'une quelconque des revendications 1 à 7 pour la détermination de la concentration en sodium, potassium ou lithium.

FIG. 1

FIG. 2

FIG. 3

FIG. 4

17

FIG. 5

FIG. 6